# EUROPEAN PATENT SPECIFICATION

(11) **EP 3 375 476 B1**
(45) Date of publication and mention of the grant of the patent: **19.02.2020**
(21) Application number: 17305274.7
(22) Date of filing: 14.03.2017
(51) Int. Cl.: A61M 16/06

(54) **RESPIRATORY MASK WITH A RESILIENT ELEMENT BETWEEN THE MASK BODY AND THE SHROUD**
ATEMMASKE MIT EINEM ELASTISCHEN ELEMENT ZWISCHEN DEM MASKENKÖRPER UND DER VORKAMMER
MASQUE RESPIRATOIRE AYANT UN ÉLÉMENT ÉLASTIQUE ENTRE LE CORPS DE MASQUE ET L'ENVELOPPE

(43) Date of publication of application: 19.09.2018
(73) Proprietor: Air Liquide Medical Systems, 92160 Antony (FR)
(72) Inventor: ALBERICI, Luca, 25030 Roncadelle (BS) (IT); BUGATTI, Ottorino, 25068 Sarezzo (Bs) (IT); FRANZONI, Mattia, 25021 Bagnolo Mella (Bs) (IT); MASSERDOTTI, Fulvio, 25075 Brescia (IT)
(74) Representative: Pittis, Olivier

(56) References cited:
- WO-A1-2012/020359
- WO-A1-2013/072799
- US-A1- 2016 129 210

## Description

The invention concerns an improved mask body for a respiratory mask, in particular a facial mask covering the nose and mouth of a patient, which is able to cooperate with the upper holding arm carried by a front shroud or retainer, located in front of the mask body, in particular for adult or pediatric uses in the treatment of respiratory conditions or diseases affecting adults, children, toddlers, infants or newborns.

Respiratory masks are commonly used for delivering non-invasive positive pressure ventilation (NPPV) or for continuous positive airway pressure (N-CPAP) therapy in disordered breathing conditions, such as obstructive sleep apnea (OSA), chronic obstructive pulmonary disease (COPD).

In both cases, the mask delivers a flow of breathable gas for or to assist in patient respiration. Examples of masks are given by EP-A-462701, EP-A-462701, EP-A-874667, EP-A-1972357 and WO-A-00/57942.

Actually, a mask typically comprises a rigid or semi-rigid hollow shell, usually made of polymer or silicone, defining a breathing chamber that receives at least a part of the patient's nose in case of a nasal mask, or both the nose and the mouth of the patient in case of a facial mask. The hollow shell or body receives the gas from a gas supply line, fixed to an inlet orifice arranged in the mask body by means of a hollow gas connector, for delivering the respiratory gas, such as air under pressure, into the breathing chamber of the shell.

A soft face-contacting cushion comes into contact with the patient's face and conforms to the various facial contours of the patient face thereby ensuring gas tightness. The cushion is usually made of soft, resilient elastomeric material, such as silicone or similar.

The mask may also include a forehead support and further a headgear for correctly positioning, maintaining and/or securing the mask on the head of a patient. The forehead support is usually carried by an upwardly projecting arm that is either directly fixed to the mask body, or to a supplementary front piece, usually called "shroud" or "retainer", that is arranged in front of the mask body, as disclosed by WO-A-0074758, EP-A-1057494 or EP-A-2708258.

When the mask is positioned on the face of a user, such as a patient, the straps of the headgear have to be tightened up so that the mask fits the facial morphology of the user.

This tightening up action may lead to a crushing of the cushion on the nasal bridge region, especially when the upper straps of the headgear of the mask are too tightened. This may lead to gas leaks and discomfort for the patient. The mask is then less efficient for delivering a gas therapy, which is not acceptable.

Further, WO-A-2013/072799 proposes a respiratory mask comprising a mask frame and a flexible cushion, a bladder assembly comprising a headgear bladder member in fluid communication with a nose bridge bladder by means of a connecting tube member. Said bladder members contain a fluid medium. The nose bridge bladder member is inflated by the fluid medium, when a pressure applies on the headgear bladder member, thereby automatically adjusting the fit of the cushion in the nose bridge region of the patient and increasing the gas tightness. The bladder assembly is arranged on the rear side of the upper arm.

In other words, the problem to be solved is to avoid the above problems in providing a mask architecture comprising a mask body and an upwardly-projecting upper arm, and further to allow an easy adjustment of the inclination of the forehead support so that the forehead support can stay in contact with the forehead without changing the position of the cushion on the face, thereby avoiding or limiting the risks of leaks and discomfort for the patient. The invention is defined by the appended claims.

The solution of the present invention concerns a respiratory mask comprising:
- a mask body comprising a gas inlet in fluid communication with an inner chamber,
- a front shroud with a flexible upwardly-projecting upper arm and comprising headgear connecting structures, and
- at least one resilient element arranged between the mask body and the flexible upper arm of the front shroud so that said at least one resilient element is squeezed between the mask body and the flexible upper arm,
characterized in that:
- the resilient element is integrally fixed, in a non-removable manner, on the peripheral outer wall of the mask body, and
- said resilient element is made of resilient material that is able to be deformed, when the flexible upper arm of the front shroud is bent or pivots toward the mask body and mechanically acts on the resilient element.

The mask according to the present invention can further comprise one or more of the following additional features:
- a resilient element is arranged on the peripheral outer wall of the mask body.
- the flexible upwardly-projecting upper arm is made of at least one plastic material, such as polycarbonate (PC), polypropylene (PP) or nylon.
- the resilient material comprises silicone.
- the resilient material has a general tubular shape, such as a cylindrical shape or any other suitable shape allowing a resilient deformation of the resilient element, when pushed toward/against the mask body by a pivoting or bending motion of the upper arm with respect of and toward the mask body.
- said at least one resilient element has a hollow structure.
- said at least one resilient element has a tubular-structure.
- said at least one resilient element has a hemi-cylinder structure.
- the front shroud comprises a central aperture coaxially arranged with the gas inlet of the mask body.
- the central aperture of the front shroud and the gas inlet of the mask body are traversed by a hollow connector.
- the hollow connector is a curved connector.
- the mask body comprises a collar element arranged around the gas inlet of the mask body, the front shroud cooperating with said collar element.
- the hollow connector is snap-fitted into the gas inlet of the mask body.
- the front shroud further comprises two lateral arms projecting laterally.
- the front shroud and/or the two lateral arms are made of at least one plastic material, i.e., polymer material, such as polycarbonate (PC), polypropylene (PP) or nylon.
- the two lateral arms comprise headgear connecting structures, such as slots, hooks and/or similar.
- it further comprises a flexible cushion fixed to the mask body.
- the front shroud is mounted in front of the mask body, i.e. surrounding, enveloping or wrapping at least a part of the peripheral outer surface of the mask body.
- the front shroud is hold integral with the mask body by the hollow connector. The front shroud is sandwiched between the hollow connector and the mask body.
- it further comprises a headgear fixed to the headgear connecting structures of the front shroud.
- the headgear comprises straps, preferably made of fabric material.
- it is a facial mask.
- the curved connector comprises an upstream and a downstream linear portion separated by an elbow portion, i.e. a bent portion.
- the downstream portion of the hollow connector is snap-fitted into the gas inlet of the mask body.
- the downstream portion of the hollow connector comprises a tubular body traversed by the inner passage and a tubular sleeve is arranged around the tubular body, said tubular sleeve comprising a free open end and a blind end, and delimits a venting passage between the tubular sleeve and the tubular body, the free open end of the tubular sleeve being arranged around the outlet orifice of the tubular body, said tubular sleeve being fixed, by the blind end, to the peripheral outer wall of the tubular body, said blind end of the tubular sleeve comprising a plurality of venting holes putting the venting passage in fluid communication with the atmosphere, preferably the venting holes are arranged in arc of circle. The venting ports, which are orifices that allow the venting of CO₂-enriched gas to the atmosphere, i.e. gas expired the patient that wears the mask. Preferably, the venting ports have a generally tronconical shape and preferably a size, such as an outlet diameter, of between about 0,3 mm and 3 mm, i.e. measured at their exit on the side of the surrounding atmosphere..
- the curved hollow connector is designed and adapted for conveying a gas, such as air under pressure (i.e. > 1 bar).
- the hollow connector has an inner diameter of the inner gas passage of between 10 mm and 30 mm, measured at the outlet orifice.
- it further comprises an anti-asphyxia system (AAS) comprising a venting port cooperating with a mobile flap. Such an AAS is known from US-A-5438981 and WO-A-00/38772. Such AAS are useful in facial masks in case of failure of the gas delivery apparatus.
- the mask body is a hollow body, also called mask shell, comprising an inner chamber and a gas inlet orifice in fluid communication with said inner chamber for allowing gas entering into said inner chamber by said inlet orifice.
- the gas inlet orifice of the mask hollow body has a diameter of about between 1 and 3 cm.
- the gas inlet orifice is arranged substantially centrally in the mask body.
- the mask body has a generally tridimensional (3D) shape, preferably a substantially triangular or trapezoidal general contour.
- when the hollow connector according to the present invention is fluidly connected to the mask hollow body, the inner chamber of the mask body is in fluid communication with the ambient atmosphere through the venting passage and the venting ports of the hollow tubular connector.
- the tubular connector comprises a tubular upstream portion for receiving and securing thereto a hose or gas line by means of a connecting piece, such as intermediary connector that is rotatable with respect to the elbow-shape tubular connector.
- when the tubular hollow connector is inserted into the gas inlet orifice of the mask body, the gas passage traversing the hollow connector is in fluid communication with the inner chamber of the mask body for delivering respiratory gas therein, whereas said inner chamber of the mask body is further in fluid communication with the venting passage and with the venting holes of the hollow connector of the invention for venting expired-gases to the ambient atmosphere, i.e. out of the mask's inner chamber.
- the flexible cushion is fixed to the mask body, for instance the cushion can be snap-fitted to the mask body, or fixed thereto by any other means, such as glued to it or even molded in one piece with the mask body.
- the cushion can be either a nasal cushion receiving only the nose of the patient (i.e. in the case of a nasal mask), or a facial cushion receiving both the nose and the mouth of the patient (i.e. in the case of a facial mask).
- the mask is preferably a facial mask covering, in use, the nose and the mouth of the patient, i.e. a facial mask.
- preferably, the cushion is a facial cushion configured and sized for coming into contact, when the mask is worn by a user, i.e. a patient, with specific regions of the user's face, comprising a nasal bridge region including lateral nose regions, a chin region, i.e. the area located under the lower lip, and cheek regions located on each lateral sizes of the nose and mouth, said cheek regions linking the nasal bridge region including lateral nose regions to the chin region. Actually, the nasal bridge region can be an area of the nose located either at the junction of bone and cartilage, or below or above said bone/cartilage junction of the nose; the chin region is expending from the lower lip to the chin, whereas the cheek regions are the areas of the face located on right and left sizes of the nose and mouth.
- the cushion comprises a cushion body delimiting a respiratory chamber comprising, on the one hand, a front aperture (i.e. front opening) adapted for receiving at least part of the patient's nose and/or mouth, in use, i.e., when the patient wears the mask, especially a facial mask receiving both the patient's nose and mouth, and/or, on the other hand, a rear aperture (i.e. rear opening) that opens into the inner chamber of the mask body, when the cushion is firmly fixed to the mask body, so that the respiratory chamber of the cushion is in fluid communication with the inner chamber of the mask body.
- the cushion has a generally tridimensional (3D) shape, preferably a 3D saddle, triangular or trapezoidal shape.
- the front aperture (i.e. front opening) of the cushion is configured and sized for receiving at least part of the patient's nose and/or mouth, in use.
- the cushion comprises at least one membrane forming a kind of flexible skirt along the cushion aperture receiving the patient's face, i.e. nose and/or mouth.
- the cushion comprises a membrane forming a flexible skirt along the cushion aperture that deforms so as to match the contours of the face of the patient thereby ensuring a gas tightness when the patient inserts his/her face, i.e. nose or nose and mouth, into the front aperture of said cushion.
- in another embodiment, the flexible cushion can comprise several membranes, e.g. two or three superimposed membranes that ensure gas tightness, the outer membrane being in contact with the patient's face, when the patient wears the mask.
- the one or several membranes are arranged around all along the periphery of the front aperture of the respiratory chamber of the cushion.
- the cushion is made of a soft flexible material, preferably silicone or similar.
- the headgear connecting structures of the front shroud comprise hooks, slots, snap-fit connectors or similar, including combination thereof.
- the front shroud comprises a upwardly-projecting upper arm and two lateral arms projecting laterally, said upwardly-projecting upper arm facing, i.e. in use, the nose and part of the forehead of the patient, and said two lateral arms projecting laterally, i.e. in use, along part of the cheeks of the patient.
- the two lateral arms and/or the upwardly-projecting upper arm are preferably integrally fixed to the front shroud, for instance molded in one-piece or glued together.
- the upwardly-projecting upper arm is also called holding arm.
- the upwardly-projecting upper arm and/or of the two lateral arms are slightly bendable toward the patient's face under the forces applied by the headgear, when said headgear is adjusted to the morphology of the patient's head.
- the two lateral arms and/or the upwardly-projecting upper arm each comprises a free distal end carrying the headgear connecting structures.
- a headgear comprising several straps is fixed to the headgear connecting structures of the two lateral arms and/or of the upwardly-projecting upper arm.
- the straps of the headgear are made of fabric and/or polymer materials, or the like.
- the upwardly-projecting upper arm and/or of the two lateral arms have an elongated shape, for example a band or ribbon shape. Of course, other shapes are possible.
- the upwardly-projecting upper arm has a length of about 2 and 8 cm.
- each lateral arms has a length of about 3 and 6 cm.
- at least a region of the upwardly-projecting upper arm and/or of the two lateral arms is configured or shaped so as to spouse, i.e. to match, the external profile, i.e. the outer contours, of the mask body, when the front shroud is positioned in contact to the mask body. Preferably, they have a curved-shape.
- the hollow connector, preferably a curved connector, is fixed to the body mask so as to be in fluid communication with one another thereby allowing gas flows to circulate, in normal use:
   - from the hollow connector to the inner chamber of the mask body during the inspiration phases of the patient, when air under pressure is delivered to the patient,
   - and vice versa, i.e. from the mask body to the hollow connector during expiration phases, when the patient exhales CO₂-enriched gases that have to be vented to the atmosphere through the venting passage and venting holes of the tubular sleeve arranged around the tubular body of the hollow connector.
- the hollow connector is fixed to and rotatable with respect to the mask body.

The invention also concerns an assembly for delivering a gas to a patient comprising a gas delivery device, such as a medical ventilator, and a respiratory mask according to the present invention. Preferably the gas delivery device is fluidly connected to the respiratory mask by means of a gas line, such as a flexible hose.

An embodiment of a facial respiratory mask according to the present invention is shown in the enclosed illustrative, but not limitative, Figures, among which:
- Figure 1 represents a general view of an embodiment of a facial mask according to the present invention,
- Figure 2 represents an exploded view of the facial mask of Figure 1, and
- Figure 3 represents a front view of another embodiment of a mask body for a respiratory mask according to the present invention,
- Figure 4 represents a side view of the mask body equipped with a front shroud of a respiratory mask according to the present invention, and
- Figure 5 is an enlarged view of encircled area A of Figure 4.

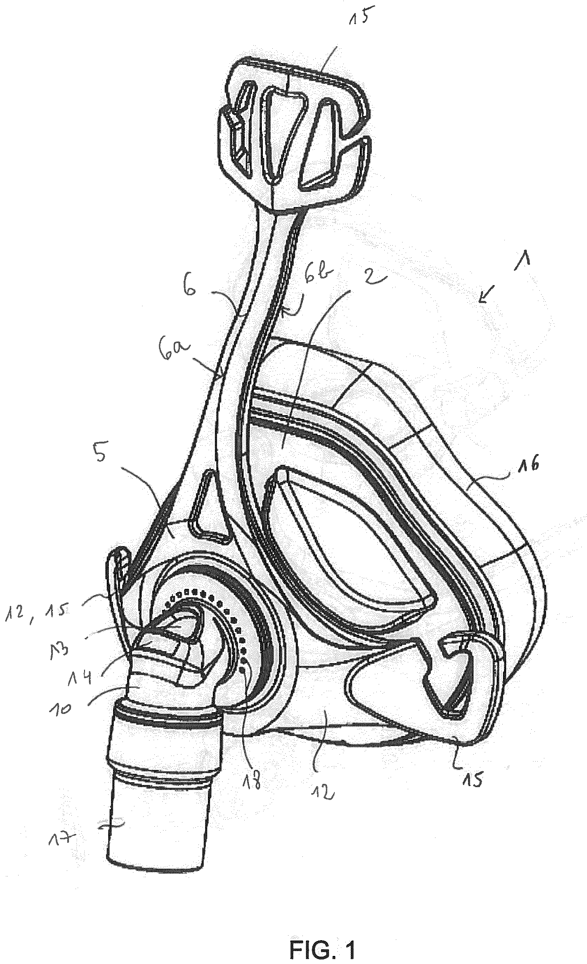

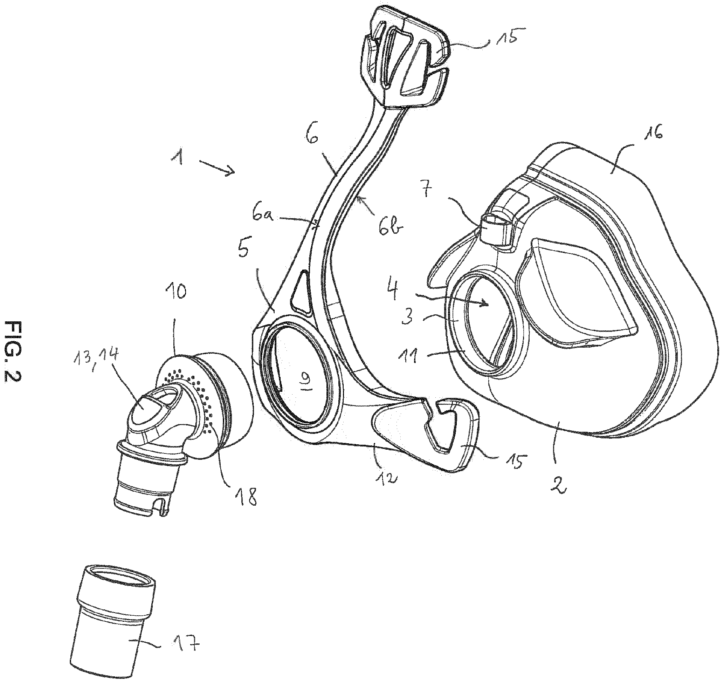

Figures 1 and 2 represent general and exploded views of an embodiment of a respiratory mask 1, namely a facial mask, according to the present invention, comprising a hollow curved connector 10, a mask body or shell 2 defining an inner chamber 4 comprising a gas inlet 3 in fluid communication with said inner chamber 4 (cf. Fig. 2), and a front shroud 5, called "retainer", "shroud" or "front piece", that is sandwiched between the tubular gas connector 10 and the mask body 2 of the mask 1.

The front shroud 5 comprises two lateral arms 12 and a flexible upper arm 6 that are integrally fixed to or molded in one-piece with the rest of the front shroud 5.

When the front shroud 5 is fixed to the mask body 2, the flexible upper arm 6 projects upwardly, i.e. about vertically, with respect to the mask body 2, whereas the two lateral arms 12 projecting laterally from said mask body 2 in opposite directions, i.e. one toward the right side of the mask 1 and the other toward the left side of the mask 1, as shown in Figures 1 and 2.

The two lateral arms 12 and the flexible upper arm 6 have free distal ends carrying headgear connecting structures 15, such as slots, hooks or the like, so as to fix a headgear thereto (not shown). A headgear typically comprises several straps of fabric and/or polymer materials, or the like, and is used for securing and maintaining the mask 1 in position on the patient's face, when the mask is used, i.e. worn by the patient.

The flexible upper arm 6 and/or of the two lateral arms 12 have typically elongated shapes, for example band or ribbon shapes, and are made of a flexible material, such as a polymer material, e.g. polycarbonate (PC), polypropylene (PP) or nylon so as to be slightly bendable toward the patient's face under the forces applied by the headgear, when said headgear is adjusted to the morphology of the patient's head. The front shroud 5 can be made of the same polymer material or of any other suitable material.

As visible in Figure 2, the shroud 5 further comprises a central aperture 9 that is traversed by part of the hollow connector 10, as illustrated in Figure 1.

Furthermore, the mask body 2 can be made from a unique piece or from several pieces or sub-units that are subsequently assembled together and made integral by means of any available fixing technique, for instance they can be glued, thermo-welded or similarly affixed together. It can be made of a polymer material that is also slightly flexible, preferably a polymer or plastic material, such as polypropylene (PP), polycarbonate (PC) and nylon, or similar.

Further, the facial mask 2 of the present invention also comprises a flexible cushion 16 that is fixed to the rear side of the mask body 2. Typically, the flexible cushion 16 is a tridimensional hollow structure forming an inner respiratory chamber 16a with a front aperture 16b adapted, i.e. conformed and sized, for receiving at least part of the nose and mouth of the patient as above explained, when the patient wears the mask, so that the patient can breathe the respiratory gas contained in the inner chamber 4 of the mask body 2 and/or in the respiratory chamber 16a of the cushion 16 as those chambers 4, 16a are in fluid communication with one another.

Indeed, the cushion 16 comprises a cushion body delimiting the respiratory chamber 16a and further comprises a rear aperture or rear opening delimited by a rear border that opens into the inner chamber 4 of the mask body 2, when the cushion 16 is firmly fixed to the mask body 2, so that gas can freely pass from the inner chamber 4 of the mask body 2 towards the respiratory chamber of the cushion 16, and vice versa.

Actually, the inner chamber 4 of the mask body 2 and the respiratory chamber 16a of the cushion 16 form together a large inner compartment wherein the patient can inspire fresh gas, such as air, during inhalation phases, and exhale CO₂-enriched gas, during expiration phases.

In other words, the respiratory chamber 16a of the cushion 16 and the inner chamber 4 of the mask body 2 are both in fluid communication with the central gas passage, i.e. the lumen, of the elbow connector 10 so that a gas flow can freely travel from said tubular connector 10 to said chambers, or vice versa.

In order to provide efficient gas tightness (i.e. seal) and/or increased comfort for the patient, the aperture 16b of the cushion 16 is delimited by a flexible membrane 16c that comes into contact with the patient's face and conforms with his/her facial morphology, when the mask 1 is worn by the patient. This membrane constitutes a kind of soft flexible skirt delimiting the front aperture of the cushion 16. Of course, depending on the embodiment, two or more superimposed membranes can also be used as using several membranes may improve gas tightness in certain circumstances, e.g. for some particular patient's morphologies.

The cushion 16 has, in the present case, a generally tridimensional (3D) trapezoidal shape, but other shapes are also possible such as triangular or saddle shapes, so as to better fit with the contours of the patient's face, especially in the nasal and mouth regions. When the facial mask 1 is worn by the patient, i.e. when the front aperture 16b of cushion 16 receives at least a part of the nose and the mouth of the patient, i.e., when the patient introduces his/her nose into the internal volume defined by the inner and the respiratory chambers, and breathes the gas contained therein or exhales CO₂-enriched gases into it, the cushion 16 and the membrane forming the peripheral border or skirt of the front aperture of the cushion 16 are in contact with particular and well defined regions of the patient's face, thereby ensuring gas tightness.

Preferably, the cushion 16 is a facial cushion configured and sized for coming into contact, when the mask is worn by a user, with at least the nasal bridge region (including lateral nose regions), the chin region, i.e. the area located under the lower lip, and the cheek regions located on both lateral sizes of the nose and mouth of the patient, said cheek regions linking the nasal bridge region including lateral nose regions to the chin region. Actually, the nasal bridge region can be an area of the nose located either at the junction of bone and cartilage, or below or above said bone/cartilage junction of the nose; the chin region is expending from the lower lip to the chin, whereas the cheek regions are the opposite areas located on right and left sizes of the nose and mouth. Of course, other embodiments and shapes of the cushion 16 are possible.

The cushion 16 (i.e., including cushion body and membrane) is preferably made of a flexible and soft material, such as silicone or the like. Preferably, the membrane and the cushion body are molded in one piece.

The cushion 16 is fixed to the mask body 2 by means of a male/female connecting system, such as male/female connections, that are configured or shaped so as to fit together for ensuring for instance a snap-fit connection, a "sandwich-type connection or the like. They can also be glued together or the like.

The gas inlet 3 of the mask body 2 is designed for fixing thereto a tubular gas connector 10 as shown in Fig. 1-2, so that the front shroud 5 is "sandwiched" and/or "squeezed" between the tubular connector 10 and the mask body 2.

The hollow connector 10 has a tubular body traversed by an inner passage comprising an inlet and outlet orifices. The tubular body of the hollow connector 10 is preferably curved so as to have a L- or elbow-shape.

In the case of a facial mask, it is wise that the connector 1 further comprises an anti-asphyxia system comprising a venting port 13 cooperating with a mobile flap 14, i.e. a flexible flap, so as to allow the patient breathing fresh air through the venting port 13 in case of failure of the gas source, such as a medical ventilator, as shown in Fig. 1-2. Such anti-asphyxia systems are known in the art, as disclosed by US-A-5438981 or WO-A-00/38772.

The upstream portion of the hollow connector 10 comprising the inlet orifice, is fed with gas, such as pressurized air, by a flexible hose (not shown) or the like conveying the gas delivered by a medical gas source, such as a medical ventilator or apparatus. The flexible hose is fluidly connected to the hollow connector 10 by means of an intermediary connector 17, i.e. a tubing element, that is preferably rotatable with respect to the hollow curved connector 10.

In the embodiment shown in Fig. 1-2, the curved connector 10 further comprises a gas venting system comprising venting passage(s) and venting orifices 18 for recovering CO₂-riched expired gases exhaled by the patient and for venting them to the ambient atmosphere. The venting holes 18 can be arranged in an array forming a circle or preferably an arc of circle (i.e., semi-circular array) as shown in Fig. 1-2.

The hollow connector 10 is entirely made of a plastic material, such as PP, PC or nylon. It is fluidly connected to the mask body 2 for feeding gas into the inner chamber 4. In other words, the gas that passes through the inner passage of the hollow curved connector 10 is subsequently delivered into the inner chamber 4 of the mask body 2 for being inhaled by a patient wearing the mask 1.

The front piece or shroud 5 comprises a central aperture 9 or opening, as shown in Fig. 2, and several headgear connecting structures 15, such as hooks and/or slots or similar, for receiving the straps of a headgear (not shown). The headgear connecting structures 15 are arranged at the free ends of the lateral arms 12 and the upper arm 6.

The gas inlet 3 of the mask body 2 and the central aperture 9 of the front shroud 5 are traversed by the hollow connector 1 as shown in Fig. 1, so that the front piece 5 is sandwiched and tightly hold between the mask body 21 and the hollow connector 10. In other words, the front piece 5 is squeezed between a collar element 11 arranged around the gas inlet 4 of the mask body 2, and the hollow curved connector 10. The hollow connector 10 can be snap-fitted into the gas inlet 4 of the mask body 2 thereby prohibiting any undesired withdrawal of the hollow connector 10 from the mask body 2.

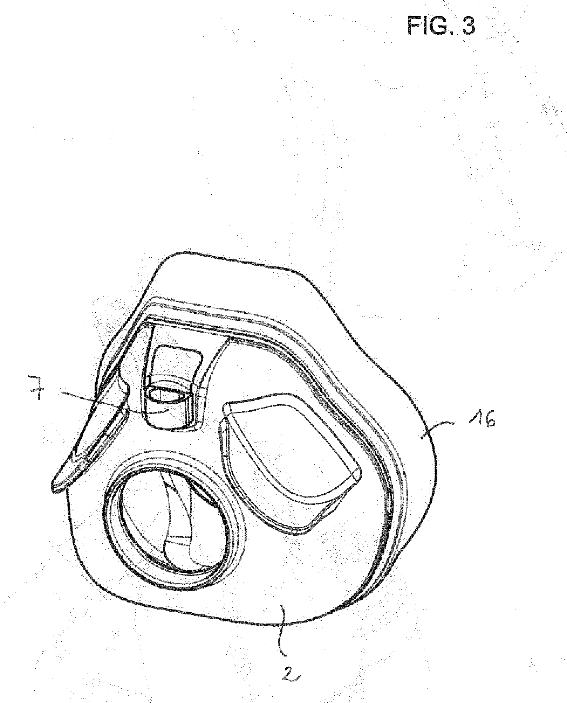

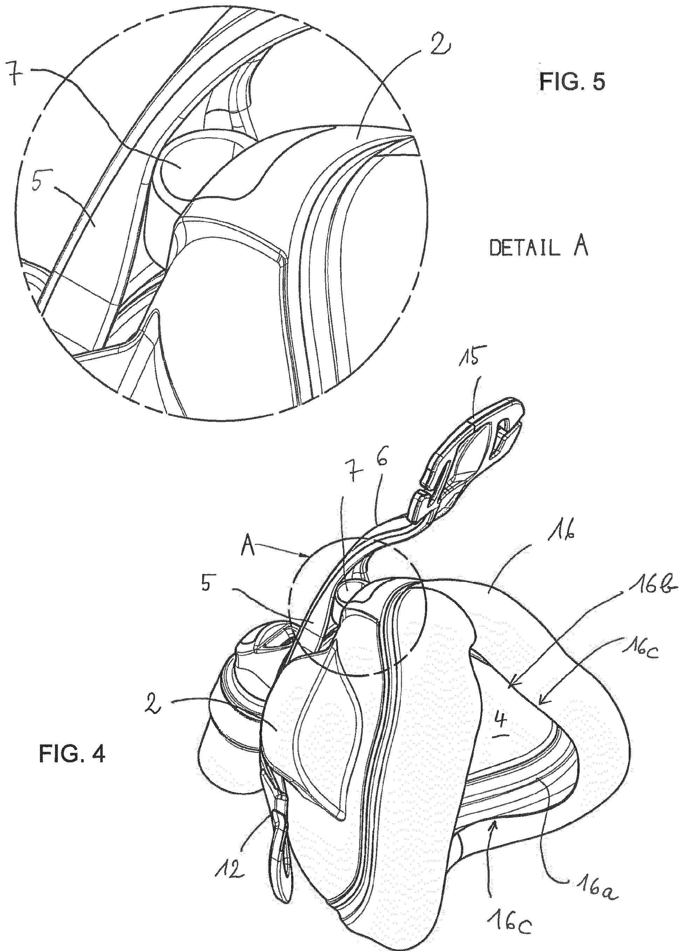

According to the present invention, as shown in Fig. 2-5, at least one (i.e. one or more) resilient element(s) 7 is(are) arranged between the mask body 2 and the flexible upper arm 6 of the front shroud 5. Said resilient element 7 is squeezed between the mask body 2 and the rear surface or side 6b of the flexible upper arm 6 of the front shroud 5, when the flexible upper arm 6 is bent toward the mask body 2, for instance when the headgear is adjusted to the facial morphology of a user.

The resilient element 7 is arranged, e. g. glued or over-molded, on the peripheral outer wall 8 of the mask body 2.

The resilient element 7 is made of resilient material, such as silicone or the like, that is able to be deformed when the flexible upper arm 6 of the front shroud 5 is bent toward the mask body 2 and mechanically acts on said resilient element 7.

Actually, the resilient element 7 acts as a soft bumper or abutment that limits the course of the upper arm 6, when it pivots toward the mask body 2, when someone pulls on the straps of the headgear for adjusting said headgear to the morphology of the patient's face.

The resilient element 7 can have a hemi-tubular shape, i.e. a hemi-cylinder, as shown in Figures 2-5, or any other suitable or equivalent shape.

In the same spirit, the resilient element 7 can be made of a unique piece or unit, such as the hemi-cylinder shown in Figures 2-5, or can be made of two or more sub-units or several pieces, that have a same shape or different shapes.

Further, the resilient element 7 can have a hollow structure, such as the hemi-cylinder of Figures 2-5, or a non-hollow structure, i.e. a solid structure, for instance made of little block or piece of resilient material.

Furthermore, the resilient element 7 is integrally fixed to the body 2, i.e. in a non-removable manner.

Generally speaking, the respiratory mask 1 of the present invention, such as a nasal or facial mask, is light, comfortable to wear, easy to position and secure on the patient's face, and provide a good tightness, i.e. gas seal.

The respiratory mask of the present invention can be used in a method for treatment of a respiratory disorder or condition affecting infant, toddler and child patients as well as adult patients, for example in non-invasive positive pressure ventilation (NPPV) or in a nasal continuous positive airway pressure (N-CPAP) therapy of sleep disordered breathing (SDB) conditions, such as, for example, obstructive sleep apnea (OSA).

## Claims

1. Respiratory mask (1) comprising:
- a mask body (2) comprising a gas inlet (3) in fluid communication with an inner chamber (4),
- a front shroud (5) with a flexible upwardly-projecting upper arm (6) comprising headgear connecting structures (15), and
- at least one resilient element (7) arranged between the mask body (2) and the flexible upper arm (6) of the front shroud (5) so that said at least one resilient element (7) is squeezed between the mask body (2) and the flexible upper arm (6),
**characterized in that**:
- the resilient element (7) is integrally fixed, in a non-removable manner, on the peripheral outer wall (8) of the mask body (2), and
- the resilient element (7) is made of resilient material that is able to be deformed, when the flexible upper arm (6) of the front shroud (5) is bent or pivots toward the mask body (2) and mechanically acts on the resilient element (7).

2. Respiratory mask according to the preceding claim, **characterized in that** the resilient element (7) comprises silicone.

3. Respiratory mask according to anyone of the preceding claims, **characterized in that** the front shroud (5) comprises a central aperture (9) coaxially arranged with the gas inlet (3) of the mask body (2).

4. Respiratory mask according to anyone of the preceding claims, **characterized in that** the central aperture (9) of the front shroud (5) and the gas inlet (3) of the mask body (2) are traversed by a hollow connector (10), preferably a curved connector (10).

5. Respiratory mask according to anyone of the preceding claims, **characterized in that** the mask body (2) comprises a collar element (11) arranged around the gas inlet (3) of the mask body (2), the front shroud (5) cooperating with said collar element (11).

6. Respiratory mask according to claim 4, **characterized in that** the hollow connector (10) is snap-fitted into the gas inlet (3) of the mask body (2).

7. Respiratory mask according to anyone of the preceding claims, **characterized in that** the front shroud (5) further comprises two lateral arms (12) projecting laterally.

8. Respiratory mask according to claim 7, **characterized in that** the two lateral arms (12) comprise headgear connecting structures (15), preferably a headgear is fixed to the headgear connecting structures (15) of the front shroud (5).

9. Respiratory mask according to anyone of the preceding claims, **characterized in that** the front shroud comprising the upwardly-projecting upper arm (6) and/or the two lateral arms, is made of at least one polymer material.

10. Respiratory mask according to anyone of the claims 1 to 8, **characterized in that** it further comprises a flexible cushion (16) fixed to the mask body (2).

11. Respiratory mask according to anyone of the preceding claims, **characterized in that** the front shroud (5) is hold integral with the mask body (2) by the hollow connector (10).

12. Respiratory mask according to anyone of the preceding claims, **characterized in that** said at least one resilient element (7) has a hollow structure, preferably a tubular-structure, more preferably a hemi-cylinder structure.

13. Respiratory mask according to anyone of the preceding claims, **characterized in that** it is a facial mask.

14. Assembly for delivering a gas to a patient comprising a gas delivery device, such as a medical ventilator, and a respiratory mask according to anyone of the preceding claims.

## Patentansprüche

1. Atemmaske (1), umfassend:
- einen Maskenkörper (2), umfassend einen Gaseinlass (3) in Fluidkommunikation mit einer internen Kammer (4),
- eine Vorderabdeckung (5) mit einem flexiblen, aufwärts ragenden, oberen Arm (6), umfassend Kopfbedeckungsanschlussstrukturen (15), und
- mindestens ein elastisches Element (7), das zwischen dem Maskenkörper (2) und dem flexiblen oberen Arm (6) der Vorderabdeckung (5) angeordnet ist, so dass das mindestens eine elastische Element (7) zwischen dem Maskenkörper (2) und dem flexiblen oberen Arm (6) eingeklemmt ist,
**dadurch gekennzeichnet, dass**:
- das elastische Element (7) einstückig, in nicht-abnehmbarer Weise auf der umlaufenden Außenwand (8) des Maskenkörpers (2) fixiert ist, und
- das elastische Element (7) aus elastischem Material hergestellt ist, das fähig ist, verformt zu werden, wenn der flexible obere Arm (6) der Vorderabdeckung (5) gebogen oder zum Maskenkörper (2) gedreht wird und auf das elastische Element (7) mechanisch einwirkt.

2. Atemmaske nach dem vorstehenden Anspruch, **dadurch gekennzeichnet, dass** das elastische Element (7) Silikon umfasst.

3. Atemmaske nach einem der vorstehenden Ansprüche, **dadurch gekennzeichnet, dass** die Vorderabdeckung (5) eine mittlere Öffnung (9) umfasst, die koaxial mit dem Gaseinlass (3) des Maskenkörpers (2) angeordnet ist.

4. Atemmaske nach einem der vorstehenden Ansprüche, **dadurch gekennzeichnet, dass** die mittlere Öffnung (9) der Vorderabdeckung (5) und der Gaseinlass (3) des Maskenkörpers (2) von einem hohlen Stutzen (10), vorzugsweise einem gekrümmten Stutzen (10) durchsetzt werden.

5. Atemmaske nach einem der vorstehenden Ansprüche, **dadurch gekennzeichnet, dass** der Maskenkörper (2) ein Kragenelement (11) umfasst, das um den Gaseinlass (3) des Maskenkörpers (2) herum angeordnet ist, wobei die Vorderabdeckung (5) mit dem Kragenelement (11) zusammenwirkt.

6. Atemmaske nach Anspruch 4, **dadurch gekennzeichnet, dass** der hohle Stutzen (10) in den Gaseinlass (3) des Maskenkörpers (2) einrastet.

7. Atemmaske nach einem der vorstehenden Ansprüche, **dadurch gekennzeichnet, dass** die Vorderabdeckung (5) weiter zwei seitliche Arme (12) umfasst, die zur Seite ragen.

8. Atemmaske nach Anspruch 7, **dadurch gekennzeichnet, dass** die zwei seitlichen Arme (12) Kopfbedeckungsanschlussstrukturen (15) umfassen, vorzugsweise eine Kopfbedeckung an den Kopfbedeckungsanschlussstrukturen (15) der Vorderabdeckung (5) fixiert wird.

9. Atemmaske nach einem der vorstehenden Ansprüche, **dadurch gekennzeichnet, dass** die Vorderabdeckung, die den aufwärts ragenden, oberen Arm (6) und/oder die zwei seitlichen Arme umfasst, aus mindestens einem Polymermaterial hergestellt ist.

10. Atemmaske nach einem der Ansprüche 1 bis 8, **dadurch gekennzeichnet, dass** sie weiter ein flexibles Kissen (16) umfasst, das an dem Maskenkörper (2) fixiert ist.

11. Atemmaske nach einem der vorstehenden Ansprüche, **dadurch gekennzeichnet, dass** die Vorderabdeckung (5) mit dem Maskenkörper (2) durch den hohlen Stutzen (10) einstückig gehalten wird.

12. Atemmaske nach einem der vorstehenden Ansprüche, **dadurch gekennzeichnet, dass** das mindestens eine elastische Element (7) eine hohle Struktur aufweist, vorzugsweise eine Schlauchstruktur, besonders bevorzugt eine Halbzylinderstruktur.

13. Atemmaske nach einem der vorstehenden Ansprüche, **dadurch gekennzeichnet, dass** sie eine Gesichtsmaske ist.

14. Anordnung zur Abgabe eines Gases an einen Patienten, umfassend eine Gasabgabevorrichtung, wie etwa ein Beatmungsgerät, und eine Atemmaske nach einem der vorstehenden Ansprüche.

## Revendications

1. Masque respiratoire (1) comprenant :
- un corps de masque (2) comprenant une entrée de gaz (3) en communication à fluide avec une chambre intérieure (4),
- une enveloppe avant (5) avec un bras supérieur souple dépassant vers le haut (6) comprenant des structures de liaison de harnais (15), et
- au moins un élément élastique (7) agencé entre le corps de masque (2) et le bras supérieur souple (6) de l'enveloppe avant (5) de sorte que ledit au moins un élément élastique (7) est serré entre le corps de masque (2) et le bras supérieur souple (6),
**caractérisé en ce que** :
- l'élément élastique (7) est fixé d'un seul tenant, d'une façon non amovible, sur la paroi extérieure périphérique (8) du corps de masque (2), et
- l'élément élastique (7) est fait de matière élastique qui est susceptible d'être déformée, quand le bras supérieur souple (6) de l'enveloppe avant (5) est plié ou pivote vers le corps de masque (2) et agit mécaniquement sur l'élément élastique (7).

2. Masque respiratoire selon la revendication précédente, **caractérisé en ce que** l'élément élastique (7) comprend de la silicone.

3. Masque respiratoire selon l'une quelconque des revendications précédentes, **caractérisé en ce que** l'enveloppe avant (5) comprend une ouverture centrale (9) agencée de façon coaxiale avec l'entrée de gaz (3) du corps de masque (2).

4. Masque respiratoire selon l'une quelconque des revendications précédentes, **caractérisé en ce que** l'ouverture centrale (9) de l'enveloppe avant (5) et l'entrée de gaz (3) du corps de masque (2) sont traversées par un raccord creux (10), de préférence un raccord incurvé (10).

5. Masque respiratoire selon l'une quelconque des revendications précédentes, **caractérisé en ce que** le corps de masque (2) comprend un élément formant collier (11) agencé autour de l'entrée de gaz (3) du corps de masque (2), l'enveloppe avant (5) coopérant avec ledit élément formant collier (11).

6. Masque respiratoire selon la revendication 4, **caractérisé en ce que** le raccord creux (10) est emboîté en force dans l'entrée de gaz (3) du corps de masque (2).

7. Masque respiratoire selon l'une quelconque des revendications précédentes, **caractérisé en ce que** l'enveloppe avant (5) comprend en outre deux bras latéraux (12) dépassant latéralement.

8. Masque respiratoire selon la revendication 7, **caractérisé en ce que** les deux bras latéraux (12) comprennent des structures de liaison de harnais (15), de préférence un harnais est fixé aux structures de liaison de harnais (15) de l'enveloppe avant (5).

9. Masque respiratoire selon l'une quelconque des revendications précédentes, **caractérisé en ce que** l'enveloppe avant comprenant le bras supérieur dépassant vers le haut (6) et/ou les deux bras latéraux, est faite d'au moins une matière polymère.

10. Masque respiratoire selon l'une quelconque des revendications 1 à 8, **caractérisé en ce qu'**il comprend en outre un coussinet souple (16) fixé au corps de masque (2).

11. Masque respiratoire selon l'une quelconque des revendications précédentes, **caractérisé en ce que** l'enveloppe avant (5) est maintenue d'un seul tenant avec le corps de masque (2) par le raccord creux (10).

12. Masque respiratoire selon l'une quelconque des revendications précédentes, **caractérisé en ce que** ledit au moins un élément élastique (7) a une structure creuse, de préférence une structure tubulaire, plus préférentiellement une structure hémicylindrique.

13. Masque respiratoire selon l'une quelconque des revendications précédentes, **caractérisé en ce que** c'est un masque facial.

14. Ensemble pour délivrer un gaz à un patient comprenant un dispositif de délivrance de gaz, comme un ventilateur médical et un masque respiratoire selon l'une quelconque des revendications précédentes.
